# EUROPÄISCHE PATENTSCHRIFT

(11) **EP 0 443 355 B1**
(45) Veröffentlichungstag und Bekanntmachung des Hinweises auf die Patenterteilung: **09.03.1994**
(21) Anmeldenummer: 91101389.4
(22) Anmeldetag: 02.02.1991
(51) Int. Cl.: C01G 19/04, C07C 51/41

(54) **Verfahren zur Herstellung von wasserfreien Zinndicarboxylatdihalogeniden**
Method for the production of anhydrous tin dicarboxylate dihalides
Procédé pour la fabrication de dicarboxylates dihalogénérés anhydres d'étain

(30) Priorität: 17.02.1990 DE 4005135
(43) Veröffentlichungstag der Anmeldung: 28.08.1991
(73) Patentinhaber: Th. Goldschmidt AG, 45127 Essen (DE)
(72) Erfinder: Ruf, Erich, Dr., W-4300 Essen (DE)

(56) Entgegenhaltungen:
- EP-A- 0 166 048
- DE-A- 2 124 876
- DE-A- 2 724 190
- DE-B- 1 299 625
- GB-A- 923 471

## Beschreibung

Aus der Literatur (Gmelin, 8. Auflage, 1975, Nr. 46, Teil C 2, Seite 221) ist die Umsetzung von Zinn(IV)-chlorid mit Essigsäure bekannt, bei der bei vorsichtigem Einengen der auf 80°C erwärmten Mischung im Vakuum Zinndiacetatdichlodrid entsteht. Bei diesem Verfahren wird das Zinndiacetatdichlorid nur in geringer Ausbeute erhalten und kommt somit für eine wirtschaftliche Herstellung nicht in Frage. Weiter ist das Arbeiten mit dem stark rauchenden, flüssigen und leicht hydrolysierbaren Zinn(IV)-chlorid umweltbelastend.

Über die Herstellung anderer Zinndiacetatdihalogenide, wie z. B. Zinndiacetatdifluorid, Zinndiacetatdibromid oder Zinndiacetatdijodid bzw. über Zinndicarboxylatdihalogenid-Verbindungen mit jeweils höhermolekularen Carboxylatresten sind in der Literatur nähere Hinweise nicht zu finden.

Der Erfindung liegt die Aufgabe zugrunde, ein Verfahren zu finden, das die Herstellung von wasserfreien Zinndicarboxylatdihalogeniden in einfacher und wirtschaftlicher Weise ermöglicht.

Diese Aufgabe wird durch das erfindungsgemäße Verfahren gelöst. Das Verfahren ist dadurch gekennzeichnet, daß man zunächst wasserfreie Zinn(II)-halogenide mit Essigsäureanhydrid im Überschuß bei Temperaturen von 20 bis 100°C unter Durchleiten von Sauerstoff oder Zusatz von Sauerstoff abgebenden Mitteln behandelt, die entstandenen Zinndiacetatdihalogenide abtrennt und diese dann mit aliphatischen Carbonsäuren oder Säureanhydriden mit mehr als vier Kohlenstoffatomen bei Temperaturen von 80 bis 150°C umsetzt und das freigesetzte Essigsäureanhydrid bzw. die freigesetzte Essigsäure unter Vakuum abdestilliert.

Vorzugsweise setzt man als aliphatische Carbonsäuren gesättigte oder ungesättigte aliphatische Carbonsäuren mit bis zu 22 Kohlenstoffatomen oder deren Säureanhydride ein.

Das erfindungsgemäße Verfahren läuft somit in zwei Stufen ab: In der ersten Stufe werden Zinndiacetatdihalogenide hergestellt, die in der zweiten Verfahrensstufe zur Bildung der gewünschten Zinndicarboxylatdihalogenide mit den entsprechenden Säureanhydriden oder Carbonsäuren umacyloxyliert werden.

### 1. Verfahrensstufe

Es ist von Vorteil, wenn man in dieser Verfahrensstufe in Gegenwart von Essigsäure als Lösungsmittel arbeitet. Durch diese Maßnahme bleiben die gebildeten Zinndiacetatdihalogenide länger in Lösung, wodurch eine schnellere Umsetzung erreicht wird.

Als Sauerstoff abgebendes Mittel verwendet man zweckmäßigerweise eine wäßrige Wasserstoffperoxidlösung. Um dabei die gewünschten wasserfreien Zinndiacetatdihalogenide zu erhalten, ist es notwendig, das über die Wasserstoffperoxidlösung eingebrachte Wasser durch Zugabe von entsprechenden Mengen an Essigsäureanhydrid in Essigsäure zu überführen. Nach einer Reaktionszeit von 2 bis 7 Stunden, während der gegebenenfalls das aufgrund der exothermen Reaktion erwärmte Reaktionsgemisch gekühlt werden kann, wird dieses dann abgekühlt, die ausgefallenen Zinndiacetatdihalogenide abfiltriert und getrocknet. Die weißen, wasserfreien Zinndiacetatdihalogenide fallen dabei in einer Ausbeute von 70 bis 90 % an.

In einer weiteren bevorzugten Maßnahme läßt man die Umsetzung der wasserfreien Zinn(II)-dihalogenide in überschüssigem Essigsäureanhydrid bei Temperaturen von 30 bis 90°C ablaufen. Da die Umsetzung des Zinndihalogenids in Essigsäureanhydrid beim Durchleiten von Sauerstoff stark exotherm ist, ist es zweckmäßig, die Reaktionsmischung durch Kühlung in diesem Temperaturbereich zu halten. Hierbei werden Ausbeuten von 90 bis 100 % erhalten.

Als Zinn(II)-halogenide werden das Chlorid, das Bromid, das Jodid und auch das Fluorid eingesetzt. Zweckmäßigerweise verwendet man das leichter zugängliche Zinn(II)-chlorid.

Die erste Stufe des erfindungsgemäßen Verfahrens weist den Vorteil auf, daß man Ausgangsprodukte einsetzen kann, die preiswert und einfach zu handhaben sind und das gewünschte Zinndiacetatdihalogenid in guter Ausbeute erhalten wird.

### 2. Verfahrensstufe

In der zweiten Stufe des erfindungsgemäßen Verfahrens werden die in der ersten Stufe erhaltenen wasserfreien Zinndiacetatdihalogenide mit aliphatischen Carbonsäuren oder Säureanhydriden mit mehr als vier Kohlenstoffatomen bei Temperaturen von 80 bis 150°C bei gleichzeitigem Abdestillieren des dabei freigesetzten Essigsäureanhydrids bzw. der freigesetzten Essigsäure unter vermindertem Druck umgesetzt. Dabei werden auf einfache Weise die entsprechenden Zinndicarboxylatdihalogenide in wasserfreier Form erhalten. In einer bevorzugten Ausführung läßt man die Umsetzung von Zinndiacetatdihalogeniden mit den aliphatischen Carbonsäuren oder Säureanhydriden mit mehr als vier Kohlenstoffatomen bei Temperaturen von 100 bis 110°C unter leichtem Vakuum ablaufen. Dabei fallen die Zinndicarboxylatdihalogenide der eingesetzten Carbonsäuren bzw. des Säureanhydrids in nahezu quantitativer Ausbeute an.

Beispiele geeigneter Carbonsäuren mit mehr als vier Kohlenstoffatomen, die für das erfindungsgemäße Verfahren geeignet sind, sind:
a) gesättigte aliphatische Carbonsäuren, wie Valeriansäure, Caprylsäure, Undecansäure, Myristinsäure, Palmitinsäure, Stearinsäure, Behensäure
b) ungesättigte aliphatische Carbonsäuren, wie Ölsäure, Linolsäure.

Beispiele geeigneter Säureanhydride sind Propionsäureanhydrid, Buttersäureanhydrid, Valeriansäureanhydrid, Laurinsäureanhydrid, Sterarinsäureanhydrid.

Die in der ersten Stufe des erfindungsgemäßen Verfahrens hergestellten wasserfreien Zinndiacetatdihalogenide können als Zinnbasisverbindungen beim Aufbringen von elektrisch leitenden und infrarotreflektierenden Schichten auf Glas- oder Keramikoberflächen eingesetzt werden. Darüber hinaus können Zinndiacetatdihalogenide als solche bzw. in Formulierungen für die chemische Konservierung von Glasoberflächen verwendet werden. Ein weiteres Einsatzgebiet für Zinndiacetatdihalogenide ist die Herstellung von zinnhaltigen Metalloxanverbindungen.

In den folgenden Beispielen wird das erfindungsgemäße Verfahren näher erläutert. Dabei werden in den Beispielen 1 bis 5 die Herstellung der wasserfreien Zinndiacetatdihalogenide (1. Stufe des erfindungsgemäßen Verfahrens) und in den Beispielen 6 bis 11 die Herstellung der Zinndicarboxylatdihalogenide (2. Stufe des erfindungsgemäßen Verfahrens) beschrieben.

### Beispiel 1

In einem 1-l-Planschliffreaktionsgefäß mit Bodenfritte, Thermometer, Kühler und Rührer werden 538,5 g Essigsäureanhydrid vorgelegt. Hierzu werden unter Rühren 189,6 g wasserfreies Zinn(II)-chlorid gegeben. In die hierbei erhaltene Aufschlämmung wird unter starkem Rühren Sauerstoff eingeleitet.

Aufgrund der exothermen Reaktion erwärmt sich die Reaktionsmischung, die durch ein Kühlbad in einem Temperaturbereich von 30 bis 50°C gehalten wird. Anstelle eines Kühlbades kann die Kühlung auch durch zeitweises Einleiten eines Inertgases, wie z. B. Argon oder Stickstoff, erreicht werden.

Das sich bildende Zinndiacetatdichlorid fällt unmittelbar aus. Die Reaktionszeit beträgt ca. 6 Stunden. Das sich hierbei gebildete Zinndiacetatdichlorid wird abfiltriert und im Vakuum ca. 0.27 kPa (ca. 2 mm Hg) getrocknet.

Ausbeute: 277,6 g Zinndiacetatdichlorid

### Beispiel 2

In einem 1-l-Planschliffreaktionsgefäß mit Bodenfritte, Thermometer, Kühler und Rührer werden 204,0 g Essigsäureanhydrid und 420 g Essigsäure vorgelegt. Zu dieser Mischung werden unter Rühren 189,6 g wasserfreies Zinn(II)-chlorid gegeben. Unter starkem Rühren wird dann Sauerstoff eingeleitet, wobei auch hier durch Kühlung die Reaktionsmischung in einem Temperaturbereich von ca. 30 bis 50°C gehalten wird. Die Reaktionszeit beträgt ca. 2 Stunden.

Es wird eine feine weiße Suspension erhalten, die in einen 1-l-Einhalskolben überführt und anschließend in einem Rotationsverdampfer bei ca. 80°C im Wasserstrahlvakuum zur Trockene eingeengt wird.

Ausbeute: 307,7 g Zinndiacetatdichlorid

### Beispiel 3

In einem 2-l-Planschliffreaktionsgefäß mit Thermometer, Kühler, Tropftrichter und Rührer werden 1 260 g Essigsäureanhydrid vorgelegt. Unter Rühren werden 758,4 g wasserfreies Zinn(II)-chlorid zugegeben.

In die hierbei erhaltene Aufschlämmung werden unter starkem Rühren langsam 194,3 g Wasserstoffperoxidlösung (70 %ig) zugetropft. Aufgrund der starken exothermen Reaktion wird die Reaktionsmischung durch Kühlung in einem Temperaturbereich von ca. 30 bis 40°C gehalten. Die Reaktionszeit beträgt ca. 7 Stunden.

Das gebildete Zinndiacetatdichlorid fällt sofort aus und wird über eine Fritte abfiltriert und viermal mit je 150 ml Chloroform gewaschen und anschließend im Vakuum ca. 0.27 kPa (ca. 2 mm Hg) getrocknet.

Ausbeute: 1 104,8 g Zinndiacetatdichlorid

### Beispiel 4

In einem 250-ml-Vierhalskolben mit Thermometer, Kühler, Rührer und Tropftrichter werden 110,2 g Essigsäureanhydrid vorgelegt. Hierzu werden 37,2 g Zinn(II)-jodid unter Rühren hinzugegeben. Danach werden unter starkem Rühren 11,4 g Wasserstoffperoxidlösung (30 %ig) zugetropft. Da die Reaktion stark exotherm ist, wird die Reaktionsmischung durch ein Kühlbad in einem Temperaturbereich von ca. 30 bis 40°C gehalten. Die Reaktionszeit beträgt ca. 2 Stunden.

Die hierbei erhaltene Zinndiacetatdijodid-Suspension wird auf -10°C abgekühlt, Zinndiacetatdijodid wird abfiltriert und fünfmal mit je ca. 100 ml Hexan gewaschen und anschließend im Vakuum ca. 0.27 kPa (ca. 2 mm Hg) getrocknet.

Ausbeute: 36,2 g Zinndiacetatdijodid

### Beispiel 5

In einem 1-l-Polyethylenkolben mit Gaseinleitungsrohr, Kühler, Rührer und Tropftrichter werden 306 g Essigsäureanhydrid vorgelegt und dann 78,4 g wasserfreies Zinn(II)-fluorid hinzugegeben.

In diese Aufschlämmung werden unter starkem Rühren 56,7 g Wasserstoffperoxidlösung (30 %ig) langsam zugetropft. Aufgrund der exothermen Reaktion erwärmt sich die Reaktionsmischung auf ca. 90°C. Die Reaktionszeit beträgt ca. 4 Stunden.

Nach beendeter Reaktion wird Essigsäure mit Hilfe eines Rotationsverdampfers im Wasserstrahlvakuum abdestilliert. Das hierbei anfallende Zinndiacetatdifluorid wird abfiltriert und fünfmal mit je 150 ml Chloroform gewaschen und anschließend im Vakuum ca. 0.27 kPa (ca. 2 mm Hg) getrocknet.

Ausbeute: 220 g Zinndiacetatdifluorid

### Beispiel 6

In einem 250-ml-Vierhalskolben mit Destillationsaufsatz, Thermometer und Rührer werden 61,5 g Zinndiacetatdichlorid und 113,8 g Stearinsäure vorgelegt. Diese Produktmischung wird unter Rühren auf ca. 100°C erhitzt. Die hierbei entstehende Essigsäure wird zunächst im Wasserstrahlvakuum und anschließend im Vakuum ca. 0.27 kPa (ca. 2 mm Hg) abdestilliert. Die Reaktionszeit beträgt ca. 4 Stunden.

Ausbeute: 148,8 g Zinndichloriddistearat

### Beispiel 7

In einem 250-ml-Vierhalskolben mit Destillationsaufsatz, Thermometer und Rührer werden 61,5 g Zinndiacetatdichlorid und 110,2 g Stearinsäureanhydrid vorgelegt. Diese Produktmischung wird unter Rühren auf ca. 100°C erhitzt. Das hierbei entstehende Essigsäureanhydrid wird zunächst im Wasserstrahlvakuum und anschließend im Vakuum ca. 0.27 kPa (ca. 2 mm Hg) abdestilliert. Die Reaktionszeit beträgt ca. 5 Stunden.

Ausbeute: 151,2 g Zinndichloriddistearat

### Beispiel 8

In einem 250-ml-Vierhalskolben mit Rührer, Destillationsaufsatz und Thermometer werden 61,5 g Zinndiacetatdichlorid und 46,5 g Capronsäure vorgelegt. Die Mischung wird unter Rühren auf 110°C erhitzt. Nach Anlegen eines leichten Vakuums wird die entstehende Essigsäure abdestilliert und in einer gekühlten Vorlage aufgefangen. Die Reaktionszeit beträgt ca. 2 Stunden.

Ausbeute: 77,0 g Zinndichloriddicapronat

### Beispiel 9

In einem 250-ml-Vierhalskolben mit Rührer, Destillationsaufsatz und Thermometer werden 61,9 g Zinndiacetatdichlorid und 58,8 g 2-Ethyl-hexansäure vorgelegt. Die Mischung wird unter Rühren und leichtem Vakuum auf 110°C erhitzt und die entstehende Essigsäure in eine gekühlte Vorlage destilliert. Die Reaktionszeit beträgt ca. 2,5 Stunden.

Ausbeute: 92,3 g Zinndichloriddi-2-ethylhexanoat

### Beispiel 10

In einem 500-ml-Vierhalskolben mit Rührer, Destillationsaufsatz und Thermometer werden 61,5 g Zinndichloriddiacetat und 80,1 g Laurinsäure vorgelegt. Ein leichtes Vakuum wird angelegt und die Mischung bei ca. 45°C aufgeschmolzen und anschließend unter Rühren weiter auf 95°C erhitzt. Die entstehende Essigsäure wird in einer gekühlten Vorlage aufgefangen. Die Reaktionszeit beträgt ca. 2 Stunden.

Ausbeute: 119,6 g Zinndichloriddilaurat

### Beispiel 11

In einem 500-ml-Vierhalskolben mit Rührer, Destillationsaufsatz und Thermometer werden 61,5 g Zinndiacetatdichlorid und 136,2 g Behensäure vorgelegt. Die Mischung wird bei ca. 80°C aufgeschmolzen und nach Anlegen eines leichten Vakuums weiter auf 120°C erhitzt. Die entstehende Essigsäure wird in einer gekühlten Vorlage aufgefangen. Die Reaktionszeit beträgt ca. 2 Stunden.

Ausbeute: 169,3 g Zinndichloriddibehenat

## Patentansprüche

1. Verfahren zur Herstellung von wasserfreien Zinndicarboxylatdihalogeniden, dadurch gekennzeichnet, daß man zunächst wasserfreie Zinn(II)-halogenide mit Essigsäureanhydrid im Überschuß bei Temperaturen von 20 bis 100°C unter Durchleiten von Sauerstoff oder Zusatz von Sauerstoff abgebenden Mitteln behandelt, die entstandenen Zinndiacetatdihalogenide abtrennt und diese dann mit aliphatischen Carbonsäuren oder Säureanhydriden mit mehr als vier Kohlenstoffatomen bei Temperaturen von 80 bis 150°C umsetzt und das freigesetzten Essigsäureanhydrid bzw. die freigesetzte Essigsäure unter Vakuum abdestilliert.

2. Verfahren nach Anspruch 1, dadurch gekennzeichnet, daß man gesättigte oder ungesättigte aliphatische Carbonsäuren mit bis zu 22 Kohlenstoffatomen oder deren Säureanhydride einsetzt.

3. Verfahren nach Anspruch 1 oder 2, dadurch gekennzeichnet, daß man in der ersten Verfahrensstufe in Gegenwart von Essigsäure als Lösungsmittel arbeitet.

4. Verfahren nach Anspruch 1, 2 oder 3, dadurch gekennzeichnet, daß man als Sauerstoff abgebendes Mittel Wasserstoffperoxid verwendet und das dadurch in das Reaktionsgemisch eingebrachte Wasser mit einer entsprechenden Menge Essigsäureanhydrid in Form von Essigsäure bindet.

5. Verfahren nach einem oder mehreren der vorhergehenden Ansprüche, dadurch gekennzeichnet, daß man in der ersten Verfahrensstufe bei Temperaturen von 30 bis 90°C und in der zweiten Verfahrensstufe bei Temperaturen von 100 bis 110°C arbeitet.

## Claims

1. Process for preparing anhydrous tin dicarboxylate dihalides, characterized in that firstly anhydrous tin(II) halides are treated with an excess of acetic anhydride at temperatures of from 20 to 100°C with the passage of oxygen or with addition of oxygen-evolving agents, the tin diacetate dihalides formed are separated off and these are then reacted with aliphatic carboxylic acids or acid anhydrides having more than four carbon atoms at temperatures of from 80 to 150°C, and the liberated acetic anhydride or the liberated acetic acid is distilled off in vacuo.

2. Process according to Claim 1, characterized in that saturated or unsaturated aliphatic carboxylic acids having up to 22 carbon atoms or their acid anhydrides are used.

3. Process according to Claim 1 or 2, characterized in that the first process stage is carried out in the presence of acetic acid as solvent.

4. Process according to Claim 1, 2 or 3, characterized in that the oxygen-evolving agent used is hydrogen peroxide and the water thereby introduced into the reaction mixture binds with a corresponding amount of acetic anhydride in the form of acetic acid.

5. Process according to one or more of the preceding claims, characterized in that the first process stage is carried out at temperatures of from 30 to 90°C and the second process stage is carried out at temperatures of from 100 to 110°C.

## Revendications

1. Procédé pour fabriquer des dicarboxylates et dihalogénures d'étain anhydres, caractérisé en ce qu'on traite d'abord des halogénures d'étain(II) anhydre avec de l'anhydride acétique en excès à des températures de 20 à 100°C par passage d'oxygène ou addition de substances cédant de l'oxygène, on sépare les diacétates et dihalogénures d'étain ainsi obtenus, et on fait réagir ces derniers avec des acides carboxyliques aliphatiques ou des anhydrides d'acides ayant plus de 4 atomes de carbone, à des températures de 80 à 150°C, l'anhydride acétique libéré ou l'acide acétique libéré étant chassé par distillation sous vide.

2. Procédé selon la revendication 1, caractérisé en ce qu'on utilise des acides carboxyliques aliphatiques saturés ou insaturés ayant jusqu'à 22 atomes de carbone, ou leurs anhydrides d'acide.

3. Procédé selon la revendication 1 ou 2, caractérisé en ce que, dans la première étape, on travaille en présence d'acide acétique servant de solvant.

4. Procédé selon la revendication 1, 2 ou 3, caractérisé en ce qu'on utilise en tant que substance cédant de l'oxygène du peroxyde d'hydrogène, et qu'on fixe l'eau ainsi incorporée dans le mélange réactionnel, avec une quantité appropriée d'anhydride acétique, sous forme d'acide acétique.

5. Procédé selon l'une ou plusieurs des revendications précédentes, caractérisé en ce que, dans la première étape, on travaille à des températures de 30 à 90°C et, dans la deuxième étape, à des températures de 100 à 110°C.
